# EUROPEAN PATENT APPLICATION

(11) **EP 3 682 850 A1**
(43) Date of publication of application: **22.07.2020**
(21) Application number: 18855628.6
(22) Date of filing: 11.09.2018
(51) Int. Cl.: A61F 2/16

(54) **INTRAOCULAR LENS INSERTION INSTRUMENT**

(30) Priority: 12.09.2017 JP 2017175051
(71) Applicant: Kowa Company, Ltd., Nagoya-shi, Aichi 460-8625 (JP)
(72) Inventor: OBA, Norio, Nagoya-shi Aichi 457-0841 (JP); KOBAYASHI, Kenichi, Nagoya-shi Aichi 457-0841 (JP)
(74) Representative: Ribeiro, James Michael
(86) International application number: PCT/JP2018/033557
(87) International publication number: WO 2019/054353

(57) **Abstract**

Provided is an intraocular lens insertion apparatus capable of stably inserting an intraocular lens while reducing stress imposed on ocular tissues. The intraocular lens insertion apparatus includes a substantially tubular insertion member configured to be inserted into an eye, an opening part provided at a tip end of the insertion member to eject an intraocular lens into the eye, and an intraocular lens push member which pushes the intraocular lens to move the intraocular lens through the insertion member and ejects the intraocular lens from the opening part into the eye, an opening direction of the opening part is tilted with respect to a direction in which the insertion member extends, a recess having a predetermined depth and extending in the extending direction is provided at an outer peripheral surface of the insertion member on an insertion member rear end side of the opening part, and the recess enlarges/contracts the opening part by elastic deformation when the insertion member is inserted into the eye and when the intraocular lens moves through the insertion member.

## Description

### [Field]

The present invention relates to an intraocular lens insertion apparatus used to insert an intraocular lens into the eye through an incision made in the eyeball.

### [Background]

In treatment of a cataract, an intraocular lens that is to be inserted as a substitute lens to replace a human opaque lens for refraction correction has become available. In an intraocular lens insertion surgery for cataract treatment, a few millimeter wound by incision (an incision) is made for example at an edge of the cornea or sclera, and the lens may be pulverized and removed through the incision by phacoemulsification, so that the intraocular lens is inserted and fixed by an intraocular lens insertion apparatus.

Stress on the ocular tissues should be smaller as the incision is smaller. Therefore, various intraocular lens insertion apparatuses have been proposed for the purpose of allowing the intraocular lens to be inserted into the eye with high operability and without damaging the incision (PTL 1).

### [Citation List]

### [Patent Literature]

[PTL 1] Japanese National Publication of International Patent Application No, 2001-517976

### [Summary]

### [Technical Problem]

However, despite the above-described feature, the shape of the tip end of the intraocular lens insertion apparatus may still differ between before and after insertion of the intraocular lens into the eye. For example, a hinge at the tip end of the intraocular lens insertion apparatus disclosed in PTL 1 having a valley fold shape before insertion of the intraocular lens may form a mountain fold after insertion of the intraocular lens. In this case, the hinge may damage the ocular tissues of the incision when the intraocular lens insertion apparatus is removed from the incision.

With the foregoing in view, it is an object of the present disclosure to provide an intraocular lens insertion apparatus capable of stably inserting an intraocular lens while reducing stress on ocular tissues.

### [Solution to Problem]

An intraocular lens insertion apparatus disclosed herein includes a substantially tubular insertion member configured to be inserted into an eye, an opening part provided at a tip end of the insertion member to eject an intraocular lens into the eye, and an intraocular lens push member which pushes the intraocular lens to move the intraocular lens through the insertion member and ejects the intraocular lens from the opening part into the eye, an opening direction of the opening part is tilted with respect to a direction in which the insertion member extends, a recess having a predetermined depth and extending in the extending direction is provided at an outer peripheral surface of the insertion member on an insertion member rear end side of the opening part, and the recess enlarges and contracts the opening part by elastic deformation when the insertion member is inserted into the eye and when the intraocular lens moves through the insertion member. With this configuration, when an operator inserts the intraocular lens into the eye with the intraocular lens insertion apparatus, the insertion member of the intraocular lens insertion apparatus can more easily be inserted into the incision made in the eye, and the intraocular lens can more smoothly move through the insertion member.

A curved surface part which connects the recess and an outer peripheral surface of the insertion member may be provided. The curved surface part may have a radius of curvature of 0.5 mm or less in a plane orthogonal to the extending direction of the insertion member. The recess of the insertion member may be kept from abutting on an inner peripheral surface of the insertion member, and the recess may have a length of 0.5 mm or more in the extending direction of the insertion member. The recess may have a depth which keeps the recess from going beyond a substantial center of a section of the insertion member in a plane orthogonal to the extending direction of the insertion member. The recess may be formed by secondary working performed after the insertion member and the opening part are formed.

### [Advantageous Effects of Invention]

According to the present disclosure, an intraocular lens insertion apparatus capable of stably inserting an intraocular lens while reducing stress imposed on ocular tissues can be provided.

### [Brief Description of Drawings]

[Fig. 1] Figs. 1(a) and 1(b) are diagrams illustrating an example of a configuration of an intraocular lens insertion apparatus according to an embodiment.
[Fig. 2] Figs. 2(a) and 2(b) are diagrams illustrating an example of a configuration of an intraocular lens according to an embodiment.
[Fig. 3] Fig. 3 is a diagram illustrating an example of a configuration of a nozzle main body according to an embodiment.
[Fig. 4] Figs. 4(a) and 4(b) are diagrams illustrating an example of a configuration of a positioning member according to an embodiment.
[Fig. 5] Figs. 5(a) and 5(b) are diagrams illustrating an example of a configuration of a plunger according to an embodiment.
[Fig. 6] Figs. 6(a) to 6(c) are diagrams illustrating an example of a configuration of a tip end of the nozzle main body according to an embodiment.
[Fig. 7] Fig. 7(a) is a schematic perspective diagram illustrating the tip end before secondary working is performed, and Fig. 7(b) is a schematic perspective diagram illustrating the tip end after secondary working is performed.
[Fig. 8] Fig. 8 is a sectional diagram illustrating the tip end taken along line A-A' in Fig. 6(c).
[Fig. 9] Figs. 9(a) to 9(c) are diagrams illustrating an example of deformation of a tip end according to an embodiment.
[Fig. 10] Fig. 10 is a diagram illustrating an example of a configuration of a tip end according to a modification.

### [Description of Embodiments]

Hereinafter, embodiments of the present invention are described with reference to drawings.

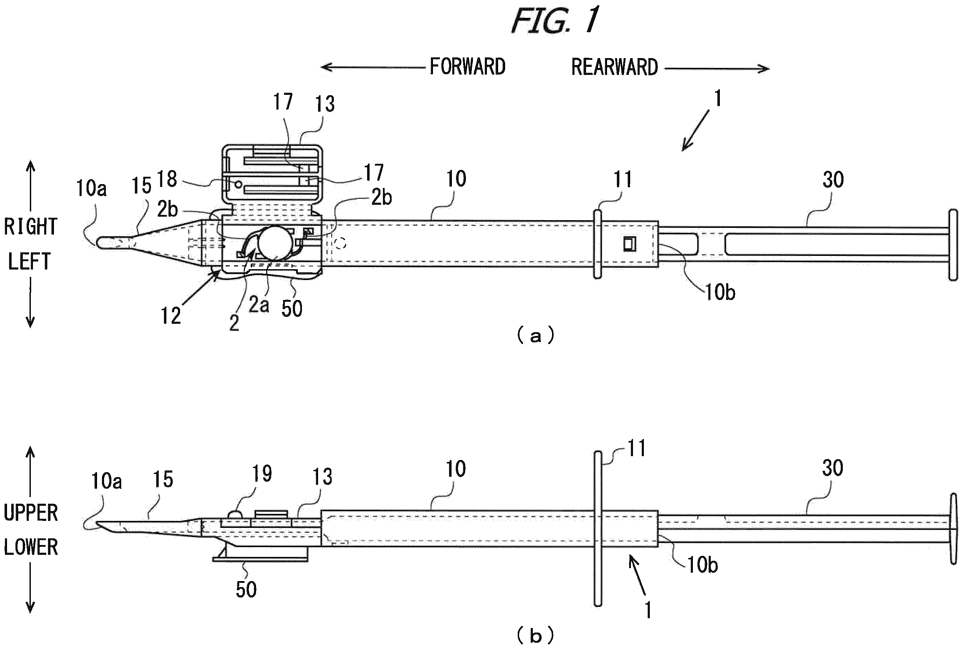

Fig. 1 schematically illustrates an outline of the configuration of an intraocular lens insertion apparatus 1 used to insert an intraocular lens into the eye. Fig. 1(a) is a plan diagram of the intraocular lens insertion apparatus 1 when a stage lid part 13 is opened, and Fig. 1(b) is a side diagram of the intraocular lens insertion apparatus 1 when the stage lid part 13 is closed. The nozzle main body 10 of the intraocular lens insertion apparatus 1 is a tubular member having a substantially rectangular cross section and has a rear end part 10b having a large opening at one end, and a tapered nozzle part 15 and a tip end part 10a at another end. As illustrated in Fig. 1(b), the tip end part 10a has a cylindrical shape with a smaller diameter, and the tip end part 10a has an end opened obliquely with respect to the extending direction of the nozzle main body 10. A plunger 30 is inserted in the nozzle main body 10 and can move back and forth therein. Note that the tip end part 10a is an example of the substantially tubular insertion member inserted into the eye, and the plunger 30 is an example of the intraocular lens push member.

In the description hereinafter, the direction from the rear end part 10b to the tip end part 10a of the nozzle main body 10 is assumed as the forward direction, the opposite direction thereto is assumed as the rearward direction, and in Fig. 1(a), the front side of the paper sheet surface is assumed as the upper side and the opposite side thereto is assumed as the lower side, while in Fig. 1(b), the front side of the paper sheet surface is assumed as the leftward direction, and the opposite side thereto is assumed as the rightward direction. In this case, the upper side corresponds to the front side of the optical axis of the lens main body 2a as will be described, the lower side corresponds to the rear side of the optical axis of the lens main body 2a, the front side corresponds to the front side in the pushing direction by the plunger 30, and the rear side corresponds to the rear side in the pushing direction by the plunger 30.

The nozzle main body 10 is provided integrally with a plate-shaped projecting holding part 11 in the vicinity of the rear end part 10b of the nozzle main body 10, and the operator can hook the finger on the projecting holding part and push the plunger 30 toward the tip end of the nozzle main body 10. A stage part 12 to which the intraocular lens 2 is set is provided behind the nozzle part 15 of the nozzle main body 10. The stage part 12 is configured to open the upper side of the nozzle main body 10 when the stage lid part 13 is opened. A positioning member 50 is attached to the stage part 12 from the lower side of the nozzle main body 10. The positioning member 50 stably holds the intraocular lens 2 in the stage part 12 before use (during transport).

More specifically, when the intraocular lens insertion apparatus 1 is manufactured, the intraocular lens 2 is set to the stage part 12 so that the front side of the optical axis is set on the upper side while the stage lid part 13 is opened and the positioning member 50 is attached to the stage part 12. The stage lid part 13 is then closed before shipment and distribution. The user dampens the lens for example with a viscoelastic material or perfusate while the stage lid part 13 is closed, then removes the positioning member 50, and then pushes the plunger 30 toward the tip end side of the nozzle main body 10.

In this way, the intraocular lens 2 is pushed by the plunger 30 to move to the nozzle part 15, and the intraocular lens 2 is ejected into the eye from the tip end part 10a. Note that the nozzle main body 10, the plunger 30, and the positioning member 50 of the intraocular lens insertion apparatus 1 are formed using a resin material such as polypropylene. Polypropylene has been proven in the field of medical apparatus and is a highly reliable material for example for its chemical resistance. The intraocular lens insertion apparatus 1 according to the embodiment is a preset type in which the intraocular lens 2 is preset in the intraocular lens insertion apparatus 1 before shipment, but the apparatus may also be a so-called separate type in which the intraocular lens 2 is set by the operator in the intraocular lens insertion apparatus 1 before operation.

A part of the stage lid part 13 is thinned, whereby a check window 17 is formed. How thin the check window 17 should be in the stage lid part 13 may be determined as appropriate on the basis of the material of the stage lid part 13 and the visibility of the intraocular lens from the check window 17. The presence of the check window 17 may probably reduce shrinkage when the stage lid part 13 is formed. The stage lid part 13 is provided with a lubricant supply hole 18 for injecting a viscoelastic material as a lubricant into the stage part 12 before the work of inserting the intraocular lens 2 into the eye. The lubricant supply hole 18 connects the outside of the stage part 12 to the intraocular lens 2 stored in the stage part 12 when the stage lid part 13 is closed.

The stage lid part 13 is provided with a guide wall 19 for guiding, to the lubricant supply hole 18, an injection member such as a needle used to inject the viscoelastic material into the space which stores the intraocular lens 2. The guide wall 19 is provided to surround at least a part of the lubricant supply hole 18, so that the operator moves the tip end of the injecting member for injecting the viscoelastic material into abutment against the guide wall 19 and further moves the tip end of the injection member to the lubricant supply hole 18. In this manner, the guide wall 19 is used as a member for guiding the injecting member for injecting the viscoelastic material to the lubricant supply hole 18.

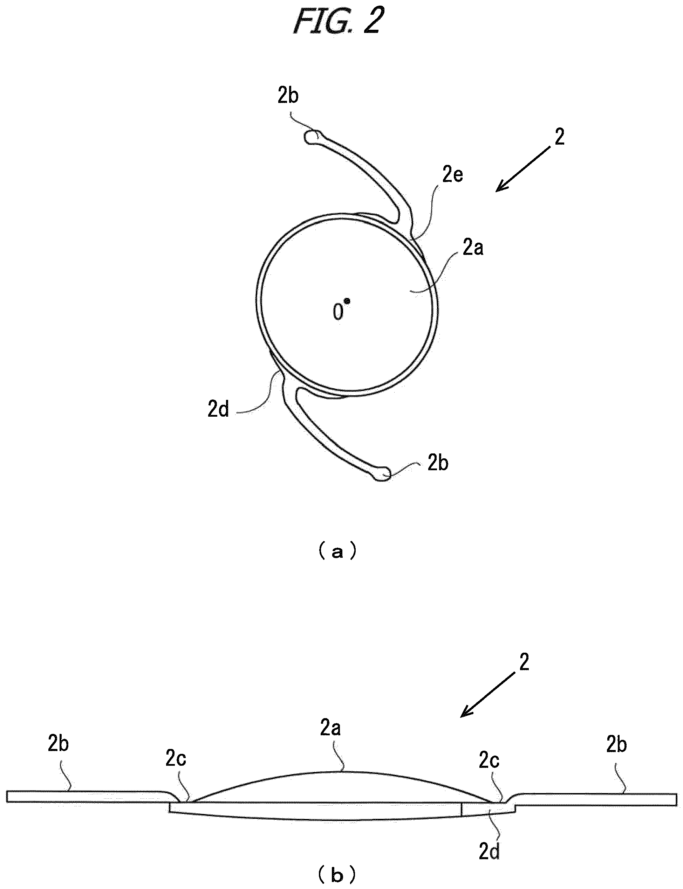

Fig. 2 is a schematic diagram illustrating an outline of the configuration of the intraocular lens 2 according to this embodiment. FIG. 2(a) is a diagram illustrating a plan view, and FIG. 2(b) is a diagram illustrating a side view. Note that the direction of the intraocular lens 2 are not the same between Figs. 2 (a) and 2(b). The intraocular lens 2 is what is called one-piece type lens including a lens main body and supports integrally formed from the same material, and the material of the lens is a flexible resin material. The intraocular lens 2 includes a lens main body 2a having a predetermined refractive power and two long flat plate-shaped supports 2b connected to the lens main body 2a to hold the lens main body 2a inside of the eyeball. The lens main body 2a and the support 2b are connected with each other through a connecting part 2d. Note that in the following description, the intraocular lens 2 according to the embodiment is a one-piece type lens, while the lens may be a three-piece type lens having a lens main body and supports made of different materials.

According to the embodiment, the intraocular lens 2 is set in the stage part 12 so that one of the two supports 2b is arranged on the rear side of the lens main body 2a and the other support 2b is arranged on the front side of the lens main body 2a in the intraocular lens insertion apparatus 1. The support arranged on the front side of the lens main body 2a is a front support, and the support arranged on the rear side of the lens main body 2a is a rear support.

The supports 2b of the intraocular lens 2 in the embodiment are roughened. In this way, the supports 2b can be prevented from sticking to the lens main body 2a when the intraocular lens 2 is folded in the nozzle main body 10.

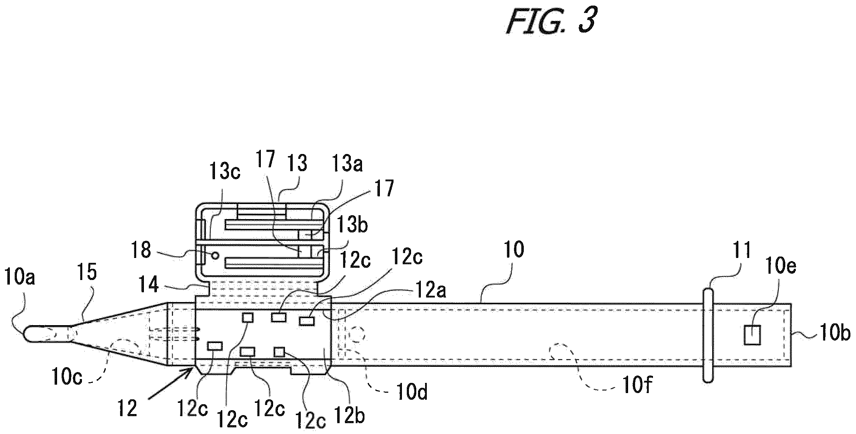

Fig. 3 is a plan view of the nozzle main body 10. As described above, the intraocular lens 2 is set in the stage part 12 in the nozzle main body 10. In this state, the intraocular lens 2 is pushed by the plunger 30 and is ejected from the tip end part 10a. Note that a through hole 10c on the tip end side and a through hole 10f on the rear end side are provided in the nozzle main body 10, and the cross-sectional shapes of the through holes change as the outer shape of the nozzle main body 10 changes. The through hole 10c forms a part of the moving path through which the intraocular lens 2 is pushed to move, and the through hole 10f is a hole into which the plunger 30 is to be inserted. When the intraocular lens 2 is ejected, the intraocular lens 2 deforms according to a change in the cross-sectional shape of the through hole 10c in the nozzle main body 10 into a folded shape, and ejected in the folded shape which is easy to enter the incision made in the eyeball of the patient.

The tip end part 10a of the nozzle main body 10 is slant as if cut off obliquely so that the upper region of the nozzle part 15 is ahead of the lower region. Note that the tip end part 10a may have a linearly obliquely cut shape as viewed from the leftward direction and rightward direction or may be slanted to have an outwardly inflated or curved shape. The obliquely cut shape of the tip end part 10a makes it easier for the operator to insert the tip end part 10a into an incision made in the eyeball of the patient compared to the case in which the tip end part 10a does not have an obliquely cut shape.

A stage groove 12a having a width slightly greater than the diameter of the lens main body 2a of the intraocular lens 2 is formed in the stage part 12. The size of the stage groove 12a in the front-back direction is set greater than the maximum width including the supports 2b, 2b which extend on both sides of the intraocular lens 2. The bottom surface of the stage groove 12a forms a set surface 12b. The set surface 12b is positioned above the height level of the bottom surface of the through hole 10f of the nozzle main body 10, and the set surface 12b and the bottom surface of the through hole 10f are connected to each other by a bottom slope 10d.

The stage part 12 and the stage lid part 13 are integrally formed. The stage lid part 13 has a size equal to the stage part 12 in the front-back direction. The stage lid part 13 is connected by a thin plate-like connecting part 14 formed by a part of the side of the stage part 12 extended toward the stage lid part 13. The connecting part 14 is formed to be bendable at the center, and the stage lid part 13 can cover and close the stage part 12 from above by bending the connecting part 14.

The surface of the stage lid part 13 facing the set surface 12b when the lid is put on is provided with ribs 13a and 13b in order to reinforce the stage lid part 13 and stabilize the position of the intraocular lens 2. A guide projection 13c is also provided as an upper guide for the plunger 30.

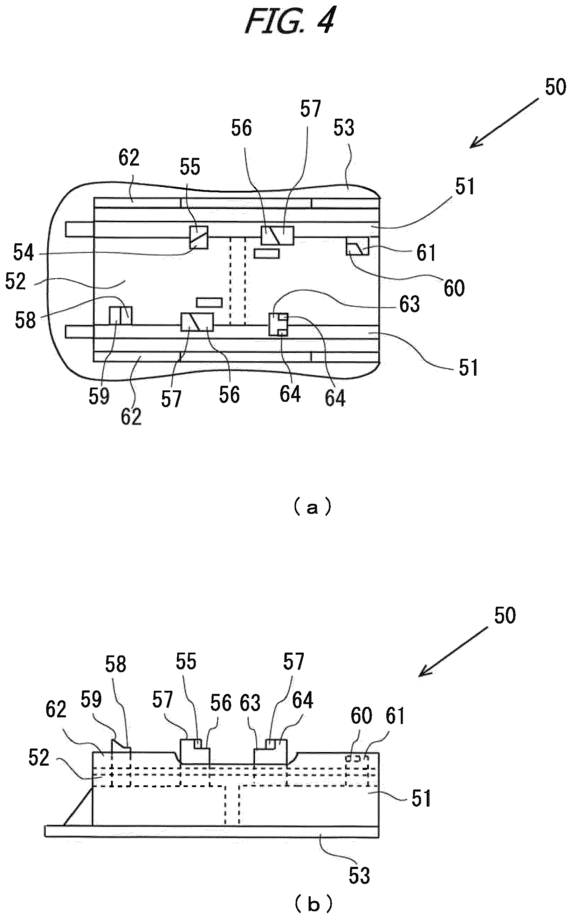

A positioning member 50 is detachably provided under the set surface 12b of the stage part 12. Fig. 4 schematically illustrates the positioning member 50. Fig. 4(a) is a plan view of the positioning member 50, and Fig. 4(b) is a left side view of the positioning member 50. The positioning member 50 is formed discretely from the nozzle main body 10 and includes a pair of sidewalls 51, 51 connected by a connecting part 52. An outwardly extending holder 53 is formed at the lower end of the sidewall 51.

First and second mounting parts 54 and 63 which protrude upwardly are formed on the inner side of the sidewalls 51, 51. A first positioning part 55 is formed to protrude at the outer peripheral side of the upper end surface of the first mounting part 54. A pair of second positioning parts 64, 64 is formed to protrude at the upper end surface of the second mounting part 63 to position the lens main body 2a and the support 2b of the intraocular lens 2. The distance between the first positioning part 55 and the second positioning parts 64, 64 is set slightly greater than the diameter of the lens main body 2a of the intraocular lens 2.

A pair of third mounting parts 56, 56 which protrude upwardly is formed inside the sidewalls 51, 51. The levels of the upper surfaces of the first mounting part 54, the second mounting part 63, and the third mounting parts 56, 56 are equal. Third positioning parts 57, 57 which project upwardly entirely over the third mounting parts 56, 56 in the leftward direction and rightward direction are formed at the outer part of the upper surfaces of the third mounting parts 56, 56. The distance between the inner sides of the third positioning parts 57, 57 is set slightly greater than the diameter of the lens main body 2a of the intraocular lens 2.

A fourth mounting part 58 on which a part of the front support of the supports 2b of the intraocular lens 2 is mounted is formed inside the sidewalls 51, 51. A fourth positioning part 59 which protrudes upwardly further above the fourth mounting part 58 is formed. A part of the front support abuts against the fourth positioning part 59. A fifth mounting part 60 on which a part of the rear support of the supports 2b of the intraocular lens 2 is mounted is formed inside the sidewalls 51, 51. A fifth positioning part 61 which protrudes upwardly further above the fifth mounting part 60 is formed. A part of the rear support abuts against the fifth positioning part 61.

As illustrated in Fig. 4(b), the levels of the upper surfaces of the fifth mounting part 60 and the fifth positioning part 61 are lower than the levels of the upper surfaces of the first to fourth mounting parts and the first to fourth positioning parts. Rotation prevention walls 62 are provided outside the sidewalls 51, 51 to prevent unwanted rotation when the positioning member 50 is detached.

The set surface 12b of the nozzle main body 10 is provided with set surface through holes 12c that pass through the set surface 12b in a thickness-wise direction. The outer shape of the set surface through hole 12c has a substantially similar shape with a slightly greater size than the shapes of the first to fifth mounting parts and the first to fifth positioning parts of the positioning member 50 as seen from above. When the positioning member 50 is mounted to the nozzle main body 10, the first to fifth mounting parts and the first to fifth positioning parts are inserted from the lower side of the set surface 12b into the set surface through holes 12c and protrude above the set surface 12b.

When the intraocular lens 2 is set to the set surface 12b, the outer peripheral bottom surface of the lens main body 2a is mounted on the upper surfaces of the first mounting part 54, the second mounting part 63, and the third mounting parts 56, 56. The lens main body 2a has its position restricted by the first positioning part 55, the second positioning parts 64, 64 and the third positioning parts 57, 57 in the horizontal direction (the horizontal direction to the set surface 12b). The two supports 2b of the intraocular lens 2 are mounted on the upper surfaces of the fourth and fifth mounting parts 58 and 60. The two supports 2b have their positions restricted in the horizontal direction by the fourth and fifth positioning parts 59 and 61.

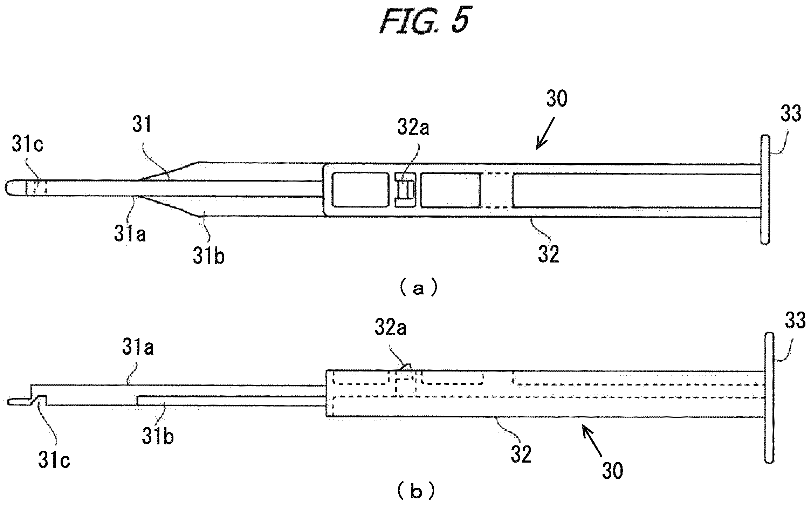

Figs. 5(a) and 5(b) schematically illustrate an outline of the configuration of the plunger 30 according to the embodiment. Fig. 5(a) is a plan view of the plunger 30, and Fig. 5(b) is a side view of the plunger 30.

The plunger 30 has a length slightly greater than the nozzle main body 10 in the front-back direction. The plunger includes a tip end side working part 31 in a generally cylindrical shape and a rear end side insertion part 32 in a generally rectangular rod shape. The working part 31 includes a cylindrical part 31a in a cylindrical shape and a flat part 31b in a thin plate shape extending in the leftward direction and rightward direction from the cylindrical part 31a.

A notch 31c is formed at the tip end of the working part 31. As illustrated in Figs. 5(a) and 5(b), the notch 31c is formed in the form of a groove which opens downward of the working part 31 and passes in the leftward direction and rightward direction. Also as illustrated in Fig. 5(b), the groove wall of the notch 31c on the tip side is formed as a downward sloping surface toward the tip end side of the working part 31. The insertion part 32 has a generally substantially H-shaped cross-section, and its horizontal and vertical sizes are set slightly smaller than the through hole 10f of the nozzle main body 10. A disk-shaped pushing plate part 33 which extends in the vertical and horizontal directions is formed at the rear end of the insertion part 32.

A claw part 32a is formed on the tip end side ahead of the center of the insertion part 32 in the front-back direction to protrude upward above the insertion part 32, and the claw part 32a can move up and down due to the elasticity of the material of the plunger 30. When the plunger 30 is inserted in the nozzle main body 10, an engaging hole 10e provided in the thickness-wise direction on the upper surface of the nozzle main body 10 illustrated in Fig. 3 and the claw part 32a are engaged, which determines the relative position between the nozzle main body 10 and the plunger 30 in the initial state. Note that the claw part 32a and the engaging hole 10e are formed so that in the engaged state, the tip end of the working part 31 is positioned behind the lens main body 2a of the intraocular lens 2 set at the stage part 12, and the rear support 2b of the lens main body 2a is arranged in a location in which the notch 31c can support the rear support from above.

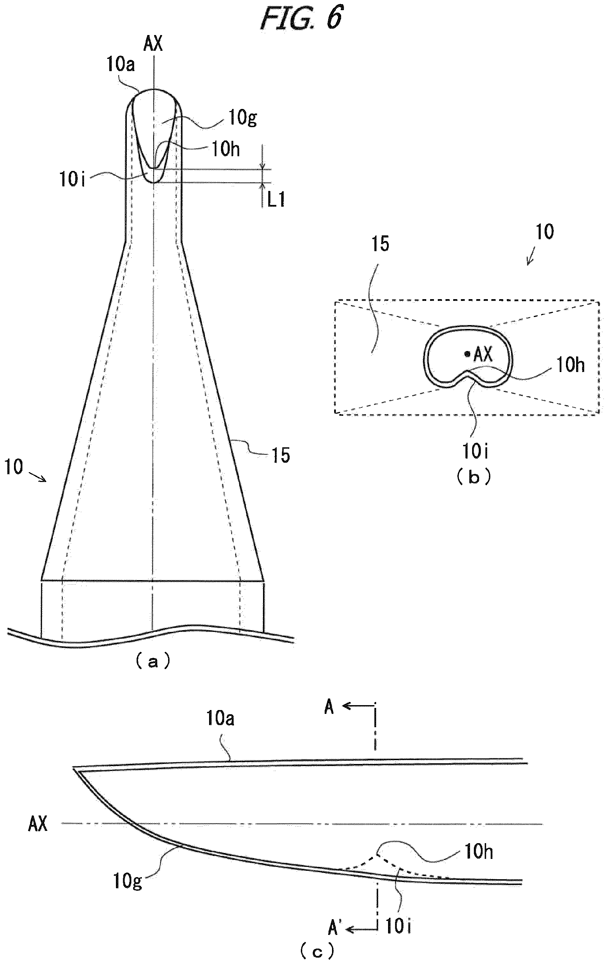

Figs. 6(a) to 6(c) illustrate the tip end part 10a and the nozzle part 15 of the nozzle main body 10. Fig. 6(a) is a plan view of the tip end part 10a and the nozzle part 15, Fig. 6(b) is a sectional view of the nozzle main body 10 taken along line A-A' in Fig. 6(c), and Fig. 6(c) is a side view of the tip end part 10a and the nozzle part 15. Note that Fig. 6(b) illustrates the nozzle part 15 by the dotted line when the nozzle main body 10 is viewed from the side of the tip end part 10a toward the rear end part 10b. As illustrated in the front view of the nozzle main body 10 in Figs. 6(a) and 6(c), the central axis of the cylindrical nozzle part 15 which extends in the front-back direction of the nozzle main body 10 is AX (the dotted double chain line in the figure). Note that the direction in which the central axis AX extends is the direction in which the tip end part 10a as an insertion member extends and matches the extending direction of the axis along which the plunger 30 is pushed.

As illustrated in Figs. 6(a) to 6(c), the edge 10h of the opening 10g of the tip end part 10a on the side of the rear end part 10b of the central axis AX is recessed toward the central axis AX. As illustrated in Fig. 6(a), according to the embodiment, the length of a recess 10i in the extending direction of the central axis AX is L1. As illustrated in Figs. 6(b) and 6(c), the recess 10i extending along the central axis AX is formed on the outer peripheral surface of the tip end part 10a. As illustrated in Fig. 6(b), the recess 10i is provided so that the distance between the part of the edge 10h through which the central axis AX passes in the plan view in Fig. 6(a) and the central axis AX is shortest.

As a method for forming the recess 10i, the edge 10h of the opening 10g deforms toward the central axis AX by secondary working performed after the opening 10g is provided at the cylindrical member as a base for the tip end part 10a for example by injection molding, in other words, secondary working performed after the insertion member and the opening part are formed, and the recess 10i illustrated in Figs. 6(a) to 6(c) is provided. One example of the secondary working is deformation by heat press fitting.

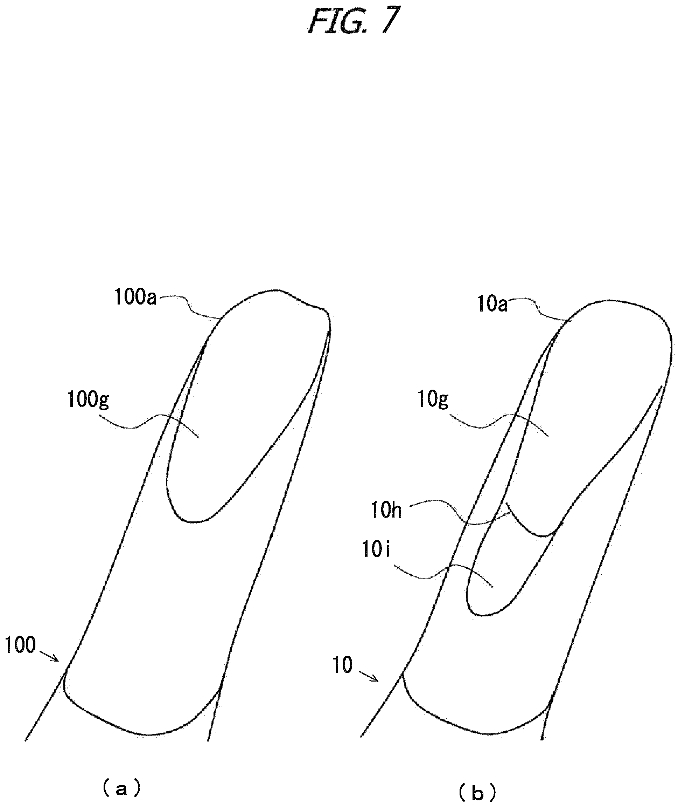

Fig. 7 (a) is a schematic perspective view of the tip end part 100a of a nozzle main body 100 before the secondary working described above is performed, and Fig. 7(b) is a schematic perspective view of the tip end part 10a after the secondary working is performed on the nozzle main body 100. Before the secondary working described above is performed, the nozzle main body 100 is formed by injection molding. As illustrated in Fig. 7 (a), although an opening 100g is provided at the tip end part 100a before the secondary working, the recess 10i is not formed. The recess 10i is formed as illustrated in Fig. 7(b) by performing secondary working such as heat press fitting to the tip end part 100a, for example, by using a mold.

Compared to the case in which the recess 10i is formed by injection molding instead of the secondary working, the recess 10i can be formed by the secondary working to have such strength that the recess 10i is unlikely to break even for the thinness and have flexibility which allows the recess 10i to restore upon deforming as the intraocular lens 2 is ejected from the opening 10g.

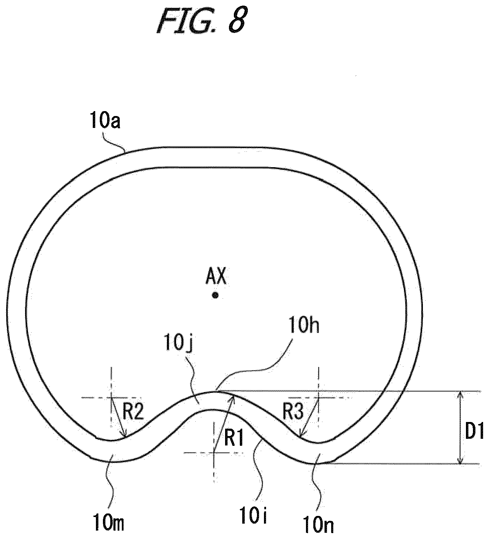

Fig. 8 is an enlarged view of the tip end part 10a in Fig. 6(b). As illustrated in Fig. 8, the recess 10i is provided with a curved surface part 10j curved toward the central axis AX. The tip end part 10a is provided with a curved surface parts 10m and 10n connecting the recess 10i and the outer peripheral surface of the tip end part 10a. The curved surface parts 10m and 10n curve toward the side opposite to the central axis AX (downward direction in the drawing).

As illustrated in Fig. 8, the radii of curvature of the curved surface parts 10j, 10m, and 10n in the plane orthogonal to the central axis AX are R1, R2, and R3, respectively. Then, possible values for R1, R2, and R3 need only be within the range from 0.1 mm to 0.5 mm. The combination of R1, R2, and R3 may be determined as appropriate. For example, in Fig. 7, it is assumed that R1, R2, and R3 satisfy R1 > R2 = R3, but the relation among the values of R1, R2, and R3 is not limited to this.

Also as illustrated in Fig. 8, the depth D1 of the recess 10i in the plane orthogonal to the central axis AX is preferably a depth which keeps the recess 10i from contacting the inner peripheral surface of the tip end part 10a. The length L1 of the recess 10i is preferably 0.5 mm or more in the direction along the central axis AX. The depth D1 is more preferably a depth which keeps the recess 10i from going beyond the central axis AX, in other words, a depth which does not go beyond an approximate center of the cross-section of the insertion member. The depth D1 and the length L1 of the recess 10i are set as described above, so that the tip end part 10a may appropriately deform when the tip end part 10a is inserted into the incision or when the intraocular lens 2 is ejected into the eye from the opening 10g. Note that the depth D1 is an example of a predetermined depth of the recess 10i, and the depth D1 may be changed to change the state of deformation of the tip end part 10a when the tip end part 10a is inserted into the incision or the state of movement of the intraocular lens 2 within the tip end part 10a.

The shape of the recess 10i of the tip end part 10a of the nozzle main body 10 when the operator inserts the intraocular lens 2 into the patient's eye using the intraocular lens insertion apparatus 1 will be described. First, before the intraocular lens 2 is stored in the intraocular lens insertion apparatus 1, the plunger 30 is inserted into the nozzle main body 10 and placed in an initial position. As described above, the positioning member 50 is mounted to the nozzle main body 10 from under the set surface 12b. In this way, the first mounting part 54, the second mounting part 63, and the third mounting parts 56, 56 of the positioning member 50 are held in a protruding state at the set surface 12b. Then, the lens main body 2a of the intraocular lens 2 is mounted and positioned on the upper surfaces of the first mounting part 54, the second mounting part 63, and the third mounting parts 56, 56 while the supports 2b are directed in the front-back direction of the nozzle main body 10. In this state, a part of the support 2b on the rear side of the intraocular lens 2 is caught and supported by the notch 31c of the plunger 30.

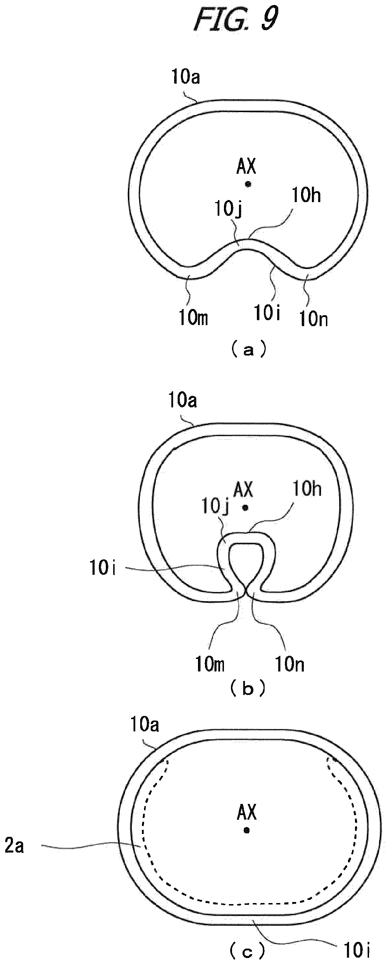

Next, the operator removes the positioning member 50 from the stage part 12. In this way, the intraocular lens 2 is set in the stage part 12. Before the tip end part 10a of the nozzle main body 10 is inserted into the incision, the recess 10i is in the state in which the curved surface parts 10m and 10n are separated from each other in a plane orthogonal to the central axis AX as illustrated in Fig. 9(a).

Then, the operator inserts the tip end part 10a of the nozzle main body 10 into an incision made in ocular tissues. Here, the tip end part 10a having an oblique opening shape can easily be inserted into the incision. At the time, as the tip end part 10a is pushed by the incision, the recess 10i shrinks in the leftward direction and rightward direction due to elastic deformation in a plane orthogonal to the central axis AX as illustrated in Fig. 9(b), and the curved surface parts 10m and 10n abut against each other, so that the opening 10g is reduced. As a result, the outer diameter of the tip end part 10a becomes smaller than that before the tip end part 10a is inserted into the incision, so that the tip end part 10a can be more easily inserted into the incision than the case in which the outer diameter of the tip end part is unchanged between before and after insertion into the incision. The curved surface part 10j moves toward the central axis AX in a plane orthogonal to the central axis AX. More specifically, since the curved surface part 10j does not protrude toward the outer peripheral side of the tip end part 10a, and the curved surface part 10j does not contact the incision when the tip end part 10a is inserted into the incision, it is less likely that the deformation of the tip end part 10a causes stress on tissues around the incision. While the tip end part 10a is inserted into the incision, the tip end part 10a is kept in the state in which the outer diameter is reduced as illustrated in Fig. 9(b).

Then, the operator operates the plunger 30 to move the intraocular lens 2 set in the stage part 12 toward the tip end part 10a. At the time, in a plane orthogonal to the central axis AX of the tip end part 10a, as the curved surface part 10j is pushed by the lens main body 2a of the intraocular lens 2 in a direction away from the central axis AX, the tip end part 10a starts to deform from the state as illustrated in Fig. 9(b), and the recess 10i is stretched in the leftward direction and rightward direction due to elastic deformation. At the time, as the curved surface part 10j moves away from the central axis AX, the curved surface parts 10m and 10n move in directions away from each other (the leftward direction and rightward direction in the figure), and the opening 10g expands. As a result, as illustrated in Fig. 9(c), the tip end part 10a deforms so that the curved surface parts 10j, 10m, and 10n become part of the cylindrical shape. Since the outer diameter of the deformed tip end part 10a as illustrated in Fig. 9(c) is larger than the outer diameter of the tip end part 10a before the tip end part 10a is inserted into the incision, a greater space can be secured for the intraocular lens 2 to pass than the case in which the outer diameter of the tip end is unchanged as the intraocular lens 2 is ejected into the eye from the tip end, so that the intraocular lens 2 can move more stably. In this way, according to the embodiment, when the tip end part 10a is inserted into an incision in the eye and when the intraocular lens 2 moves through the tip end part 10a, the recess 10i provided at the tip end part 10a shrinks/expands by elastic deformation to reduce/increase the opening 10g, so that stress on the ocular tissues of the incision can be reduced and the intraocular lens can stably be inserted.

When the recess 10i deforms as illustrated in Fig. 9(c), the tip end part 10a is pushed by the incision, so that a restoring force to bring back the recess 10i into the shape as illustrated in Fig. 9(b) is exerted. Meanwhile, when the intraocular lens 2 moves through the tip end part 10a, the lens main body 2a is curved in the tip end part 10a as illustrated in Fig. 9(c), and therefore a restoring force to bring back the lens main body 2a into the flat plate shape as illustrated in Fig. 2(b) is exerted. More specifically, when the intraocular lens 2 moves through the tip end part 10a, the restoring force of the recess 10i against the restoring force of the lens main body 2a of the intraocular lens 2 acts on the lens main body 2a.

Meanwhile, the presence of the recess 10i causes a restoring force to bring back the tip end part 10a into the shape as illustrated in Fig. 9(a) from the shape as illustrated in Fig. 9(b). More specifically, the restoring force acts to ensure the nozzle, through which the lens passes, to have a greater inner diameter cross-sectional area. This increases the cross-sectional area in the range in which the lens passes and reduces inequalities in extrusion resistance. Since the plunger collides against the recess, the collision serves as resistance and there may be less fluctuations in extrusion load. Consequently, it can be considered that the likelihood of the lens main body 2a shooting out of the opening 10g of the tip end part 10a at a speed unexpected by the operator or a so-called rocket launch may be reduced. As a result, when the intraocular lens 2 moves through the tip end part 10a, it is less likely that the tip end part 10a suddenly deforms into the shape as illustrated in Fig. 9(c) and that the deformation of the tip end part 10a imposes excessive stress on the tissues of the incision.

It can be considered that when the recess 10i deforms as described above, the restoring force of the recess 10i is greater than the restoring force of the part other than the recess 10i of the tip end part 10a. Therefore, when for example the recess 10i deforms so that the curved surface parts 10j, 10m, and 10n become part of the cylindrical shape as illustrated in Fig. 9 (c), the recess 10i regains the shape when the nozzle is inserted into the eye as illustrated in Fig. 9(b). When the recess 10i deforms as described above, the stress acting on the tip end part 10a is not concentrated at the recess 10i, and the entire tip end part 10a deforms. Therefore, it can be considered that the recess 10i is unlikely to plastically deform beyond the elastic limit in the elastic deformation of the recess 10i.

When the tip end part 10a deforms as illustrated in Fig. 9(c), the resistance of the lens due to the restoring force of the tip end part 10a is transmitted from the lens main body 2a to the plunger 30, so that the operator can sense the restoring force of the tip end part 10a through the plunger 30 when the lens main body 2a is moved to the tip end part 10a by the plunger 30. Therefore, the restoring force of the tip end part 10a can also be used as a cue to inform the operator operating the plunger 30 of the arrival of the lens main body 2a at the tip end part 10a.

When the intraocular lens 2 is ejected into the eye from the opening 10g of the tip end part 10a, the tip end part 10a is pushed by the incision, and therefore the shape as illustrated in Fig. 9(c) returns to the shape as illustrated in Fig. 9(b). Then, when the tip end part 10a is removed from the incision, the tip end part 10a returns to the shape as illustrated in Fig. 9(a) from the shape as illustrated in Fig. 9(b). At the time, since the tip end part 10a deforms according to the size of the opening of the incision, it is unlikely that the tissues of the incision are stressed such as, for example the opening of the incision is widened by the deformation of the tip end part 10a.

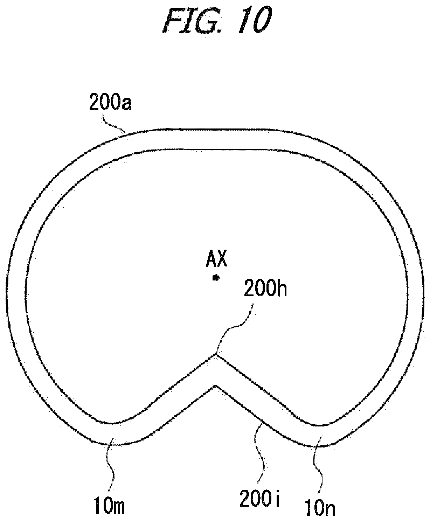

Although the embodiment has been described, the features of the tip end part of the intraocular lens insertion apparatus or other elements are not limited by the above described embodiment, and various changes can be made within the range consistent with the technical idea of the present invention. For example, as for the radii of curvature R2 and R3 of the curved surface parts 10m and 10n in Fig. 8, the radius of curvature of one of the curved surface parts passed by the rear support of the intraocular lens 2 may be set smaller. In this way, spaces with different sizes are provided in the leftward direction and rightward direction of the tip end part 10a, and when the intraocular lens 2 is inserted into the eye, pressure exerted on the lens main body 2a passing through the larger space is reduced, so that increase or decrease in the extrusion resistance is reduced, and the rear support passes through the smaller space. This configuration can control the posture of the intraocular lens 2 until the insertion of the intraocular lens 2 is completed. Instead of the above-described tip end part 10a having the shape as illustrated in Fig. 9(a), a tip end part 200a having a recess 200i in the shape as illustrated in Fig. 10 (the part from 10m to 10n through 200i in the figure) may be used. The recess 200i has a folded part 200h corresponding to the part 10j of the tip end part 10a in Fig. 9 (a). When the recess 200i is used, similarly to the above-described embodiment, the recess 200i can deform when the tip end part 200a is inserted into the incision and when the intraocular lens 2 is moved through the tip end part 200a, so that the intraocular lens 2 can smoothly be inserted into the eye by the plunger 30 while reducing the likelihood of stress imposed on the tissues of the incision.

Embodiments of the intraocular lens insertion apparatus using the present invention is not limited to the above-described embodiment and the invention can also be applied to intraocular lenses and intraocular lens insertion apparatuses for example as disclosed in Japanese Laid-open Patent Publication No. 2017-445. More specifically, the intraocular lens is not limited to the one-piece lens illustrated in Fig. 2 and may be a three-piece lens as disclosed in the Japanese Laid-open Patent Publication No. 2017-445. The plunger is not limited to the shape illustrated in Fig. 3 and may be a plunger in a shape as disclosed in Japanese Laid-open Patent Publication No. 2017-445. The deformed shape of the lens at the time of insertion may be not only a so-called valley fold shape obtained by rounding the lens at the bottom while the top is open, as illustrated in Fig. 9(C), but also a so-called mountain fold shape obtained by turning the lens upside down and then rounding the lens at the top while the bottom is open.

### [Reference Signs List]

- 1: Intraocular lens insertion apparatus
- 10a, 200a: Tip end part
- 10g: Opening
- 10i, 200i: Recess
- 10j, 10m, 10n: Curved surface part

## Claims

1. An intraocular lens insertion apparatus, comprising:
a substantially tubular insertion member configured to be inserted into an eye;
an opening part provided at a tip end of the insertion member to eject an intraocular lens into the eye; and
an intraocular lens push member which pushes the intraocular lens to move the intraocular lens through the insertion member and ejects the intraocular lens from the opening part into the eye,
an opening direction of the opening part being tilted with respect to a direction in which the insertion member extends,
a recess having a predetermined depth and extending in the extending direction being provided at an outer peripheral surface of the insertion member on an insertion member rear end side of the opening part,
the recess enlarging and contracting the opening part by elastic deformation when the insertion member is inserted into the eye and when the intraocular lens moves through the insertion member.

2. The intraocular lens insertion apparatus according to claim 1, further comprising a curved surface part which connects the recess and an outer peripheral surface of the insertion member.

3. The intraocular lens insertion apparatus according to claim 2, wherein the curved surface part has a radius of curvature of 0.5 mm or less in a plane orthogonal to the extending direction of the insertion member.

4. The intraocular lens insertion apparatus according to claim 3, wherein the recess of the insertion member is kept from abutting on an inner peripheral surface of the insertion member and the recess has a length of 0.5 mm or more in the extending direction.

5. The intraocular lens insertion apparatus according to claim 4, wherein the recess has a depth which keeps the recess from going beyond a substantial center of a section of the insertion member in a plane orthogonal to the extending direction of the insertion member.

6. The intraocular lens insertion apparatus according to any one of claims 1 to 5, wherein the recess is formed by secondary working performed after the insertion member and the opening part are formed.
